# EUROPÄISCHE PATENTANMELDUNG

(11) **EP 3 632 894 A1**
(43) Veröffentlichungstag der Anmeldung: **08.04.2020**
(21) Anmeldenummer: 18197945.1
(22) Anmeldetag: 01.10.2018
(51) Int. Cl.: C07C 319/20, C07C 323/58, C07C 323/60

(54) **NEBENPRODUKTARME HERSTELLUNG VON METHIONIN AUS METHIONINNITRIL**

(71) Anmelder: Evonik Operations GmbH, 45128 Essen (DE)
(72) Erfinder: ROST, Daniel, 68163 Mannheim (DE); REUS, Christian, 63579 Freigericht (DE); BILZ, Jürgen, 63579 Freigericht (DE); KORFER, Martin, 63796 Kahl (DE); HASSEBERG, Hans -Albrecht, 63584 Gründau-Lieblos (DE)
(74) Vertreter: Evonik Patent Association

(57) **Zusammenfassung**

Die Erfindung betrifft ein Verfahren zur Herstellung von Methioninamid durch Hydrolyse von Methioninnitril in Gegenwart von 0,5 bis 1,5 Moleq eines Ketons als carbonylhaltigen Katalysator und von 0,03 bis 0,10 Moleq KOH als basischer Katalysator jeweils bezogen auf 1 Moleq Methioninnitril sowie ein damit verbundenes Verfahren zur Herstellung von Methionin durch Hydrolyse des so gewonnenen Methioninamids.

## Beschreibung

DL-Methionin ist eine essenzielle Aminosäure, die mit der Nahrung aufgenommen werden muss. Als Futtermittelzusatz trägt sie zu einer effizienten, gesunden und umweltschonenden Ernährung von landwirtschaftlichen Nutztieren, insbesondere von Geflügel und Schweinen, bei. Sie ist damit auch ein wichtiger Baustein, wenn es um die nachhaltige Versorgung einer wachsenden Weltbevölkerung mit tierischem Protein geht. Somit kommt einer kostengünstigen und auch großindustriell gut durch führbaren Synthesemethode für DL-Methionin eine hohe Bedeutung zu.

### Stand der Technik:

Im industriellen Maßstab wird Methionin chemisch über die Bucherer-Bergs-Reaktion hergestellt, die eine Variante der Strecker-Synthese darstellt. Dabei werden die Ausgangssubstanzen 3-Methylthiopropanal (= 3-Methylmercaptopropionaldehyd, MMP; hergestellt aus 2-Propenal und Methylmercaptan), Blausäure (Cyanwasserstoff), Ammoniak und Kohlendioxid zum 5-(2-Methylmercaptoethyl)-hydantoin (Methioninhydantoin) umgesetzt. Die Hydrolyse des Hydantoins erfordert harsche Bedingungen und stöchiometrische Mengen an einer Base, meist Natriumhydroxid oder Kaliumhydroxid bzw. Kaliumcarbonat. Nach einem altbekannten Verfahren wird Methionin im Hydrolysat durch Neutralisation mit Schwefelsäure aus seinem Natriumsalz freigesetzt, welches als Präzipitat aus der Natriumsulfat enthaltenden Mutterlauge abfiltriert werden kann. Das Koppelprodukt Natriumsulfat muss anderweitig verwertet oder entsorgt werden.
Nach dem bekannten Degussa-Kaliumcarbonatkreislaufverfahren wird Methionin schließlich durch Behandlung des Hydrolysats mit Kohlendioxid aus seinem Kaliumsalz freigesetzt, wobei das Methionin-Präzipitat aus der Kaliumcarbonat und Kaliumhydrogencarbonat enthaltenden Mutterlauge abfiltriert werden kann (US 5,770,769). Letzteres kann zwar zurückgewonnen werden, doch ist dafür ein Kreislauf einer großen Menge salzhaltiger Lösung erforderlich.
Bedingt durch den mit der Zeit ansteigenden Nebenproduktpegel in der zu recyclierenden wässrigen Mutterlauge ist es notwendig nicht unerhebliche Mengen der Mutterlauge aus dem Verfahren auszuschleusen, was den zusätzlichen Aufwand einer Aufarbeitung oder Entsorgung mit sich bringt.
Zum anderen sind die Bedingungen der Hydantoinbildung und der Hydantoinhydrolyse mit Temperaturen von bis zu über 200 °C harsch und energieintensiv, so dass weiterhin Bedarf an einer großindustriell durchführbaren Methode bestand, die die genannten Nachteile nicht oder kaum mehr aufweist.

Eine bekannte Alternative zur Bucherer-Bergs-Reaktion stellt der Herstellungsweg via Methioninnitril, dem Aminonitril von 3-Methylmercaptopropionaldehyd (MMP-AN), und dessen anschließende Verseifung zu Methionin mit Hilfe stöchiometrischer Mengen Kalilauge oder Schwefelsäure nach Strecker dar. Aber auch diese Route macht der relativ hohe stöchiometrische Salzabfall, der durch die ebenfalls nötige Neutralisation bedingt ist, unattraktiv für ein großindustrielles Herstellungsverfahren.
Alternativ können die Aminonitrile wie zum Beispiel Methioninnitril in Gegenwart eines Ketons wie zum Beispiel Aceton und einer starken Hydroxidbase wie zum Beispiel Natriumhydroxid als Katalysator mit hoher Selektivität schon bei Raumtemperatur in die entsprechenden alpha-Aminoamide umgewandelt werden (z.B. Bull. Soc. Chim. Fr. 1978, 3-4, II-177 bzw. DE2637204.). In DE2637204 werden im Falle der Methioninamidbildung noch 1,25 Moleq (Molequivalente) NaOH und 72 Moleq Aceton benötigt. Die Ausbeute beträgt nach 2 Stunden bei 22 bis 32 °C 95,7% Methioninamid. EP0228938 (entspricht E 41148 B) benötigt für diese Reaktionsstufe nur noch 0,2 Moleq NaOH und 0,2 Moleq Aceton und ca. 1 Stunde bei 30°C (Beispiel 1).
Eine anschließende Verseifung des nach solchen Verfahren gebildeten Methioninamids mittels Alkalien liefert unter vergleichsweise milden Bedingungen von z.B. 80 - 120°C das Alkalimetallsalz des Methionins (EP0228938), das direkt als Futtermitteladditiv verwendet werden kann (WO94/08957 A1) oder aus dem wiederum durch Neutralisation mit Säure Methionin erst freigesetzt werden muss unter Bildung entsprechender Salze als unerwünschtem Koppelprodukt (Schema 1). Die Ausbeuten an Methioninamid bzw. Methionin liegen bei ca. 95-97 % und sind damit zwar hoch jedoch nicht quantitativ.

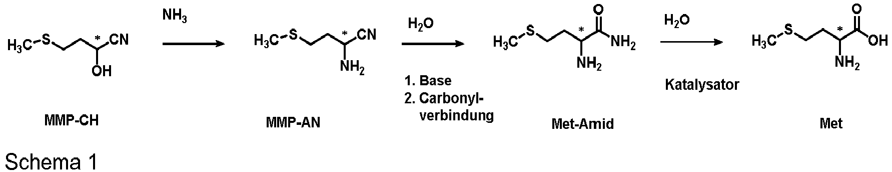

Aber auch wenn die anschließende Verseifung des Amides direkt zur Carbonsäure über ein Neutralverseifungsverfahren mit einem heterogenen Katalysator (z.B. TiO₂) angewendet wird (z.B. DE 60127538 T2, Adisseo), so ist diese Route dennoch nicht salzfrei, da in der Regel 10-20 mol% einer starken Base wie z.B. NaOH in der Amidbildungsreaktion eingesetzt werden müssen. Diese Base muss durch physikalisch-chemische Methoden wie z.B. lonenaustauscherharze wieder abgetrennt werden, um nicht das entsprechende Methioninsalz wie zum Beispiel Natriummethioninat als Beiprodukt zu erhalten.
Eine Variante der Keton-katalysierten Umsetzung ist mit heterogenen Polymer-gebundenen Carbonylverbindungen beschrieben (US 4,851,576). Nachteilig ist jedoch, dass die Polymere nicht käuflich verfügbar sind und durch Nebenreaktionen mit der Zeit deutlich an Aktivität verlieren, sodass sie aufwendig regeneriert werden müssen.
Alternativ ist die Verwendung von polymer gebundenen Hydroxidbasen bekannt (z.B. FR2785609). Diese haben jedoch den Nachteil, dass sie ebenfalls häufig regeneriert werden müssen, was wiederum zu Salzabfall führt.

Darüber hinaus sind im Stand der Technik mehrere Verfahrensvarianten für andere Aminosäureamide außer Methioninamid offenbart, die sich auch die carbonylkatalysierte alkalische Hydrolyse der betreffenden Aminonitrile zu Nutze machen.

JP2001-199947A offenbart ein Verfahren zur Hydrolyse von Aminosäurenitril zu Aminosäureamiden bzw. Aminosäuren. Dabei wird zunächst aus einem Cyanhydrin mit Ammoniak ein entsprechendes Aminonitril hergestellt. Dabei werden Alkalimetallhydroxide wie Natriumhydroxid oder Kaliumhydroxid in einem Vorzugsbereich von 0,01-0,10 Moleq pro ein Moleq Cyanhydrin und Methylethylketon oder Aceton als Carbonylkatalysator verwendet und in einem Temperaturbereich von 0-20 °C zur Reaktion gebracht. Alaninamid, Valinamid und Phenylglycinamid wurden auf diese Weise erhalten. Die Reaktion mit Methioninnitril wurde dort nicht untersucht.

JP 2001- 247529 A offenbart ebenfalls ein Verfahren zur Hydrolyse von Aminosäurenitril zu Aminosäureamiden. Methioninamid wird nicht erwähnt. Im Beispiel beschrieben ist nur ein Verfahren zur Herstelllung von tert. Leucinamid. Dabei werden Alkalimetallhydroxide wie Natriumhydroxid oder Kaliumhydroxid vorzugsweise in einem überaus breiten Vorzugsbereich von 0,01-10 Moleq (0,5 Moleq im Beispiel) pro ein Moleq Aminonitril und niedermolekulare Ketone, insbesondere Aceton als Carbonylkatalysator verwendet, bei Temperaturen von ca. 20 - 30°C und sehr langen Reaktionszeiten von 1-100 Stunden. Im Falle von tert.Leucinamid wurden auf diese Weise jedoch nur 83 bis 91% Ausbeute bzw. 98% Rohausbeute erhalten. Ein Verfahren zur Herstellung von Methioninamid ist nicht offenbart. Der relativ hohe Basenanteil von z.B. 0,5 Moleq NaOH erfordert wiederum einen relativ hohen Säureanteil, der zur Neutralisation und Isolierung von neutralem Methionin gebraucht wird. Das führt zu erheblichem Anfall von Salz als Beiprodukt, was unter ökonomischen wie ökologischen Gesichtspunkten nachteilig ist.

JP5613162B A offenbart schließlich ein Verfahren zur Herstellung des Salzes von 2-Aminobuttersäureamid mit einer anorganischen Säure durch Hydrolyse von 2-Aminobuttersäurenitril mittels einer starken anorganischen Base und einem Keton (Aceton oder Methylethylketon) als Lösungsmittel und damit in großer Menge. Dabei werden Alkalimetallhydroxide wie Natriumhydroxid oder Kaliumhydroxid in einem Vorzugsbereich von 0,005-0,10 Mol pro ein Mol Cyanhydrin und Methylethylketon oder Aceton als Carbonylkatalysator verwendet und bei einer Temperatur von 20 °C 7 Stunden lang (Beispiele) zur Reaktion gebracht. Am Ende wird durch Zugabe einer starken Mineralsäurelösung das Mineralsäuresalz des 2-Aminobuttersäureamids erzeugt. Auch diese Variante ist aufgrund des hohen Anfalls von Salz als Beiprodukt nachteilig.

CN102070473 A offenbart schließlich ein Verfahren zur Herstellung von 2-Amino-3-methylbuttersäureamid (Valinamid) durch Hydrolyse von 2- Amino-3-methyl-buttersäurenitril (Valinnitril) mittels einer starken anorganischen Base und einem Keton (Aceton oder Methylethylketon) als Carbonylkatalysator. Dabei werden Natriumhydroxid, Kaliumhydroxid oder Bariumhydroxid im Gewichtsverhältnis von 0,01-1,0 :1 und bevorzugt 0,05-0,50 :1 bezogen auf die Menge an Keton und das Keton im Verhältnis von 0,5-2,0 Moleq pro 1 Moleq Valinnitril und bei einer Temperatur von -10 bis 10 °C 5-8 Stunden lang (Beispiele) zur Reaktion gebracht. Am Ende wird durch Extraktion mit organischen Lösungsmittel (Chloroform) das Valinamid aus der wässrigen Salzlösung entfernt, was mit verringerten Ausbeuten von nur ca. 63-85 % einhergeht. Auch diese Variante ist aufgrund des hohen Anfalls von Salz als Beiprodukt und der Verwendung von organischem Lösungsmittel nachteilig.

Wie die Erfinder beim Nacharbeiten des Stands der Technik feststellen mussten, hat die carbonylkatalysierte basische Hydrolyse von Methioninnitril den Nachteil, dass sich durch die Alkalilabilität des eingesetzten Methioninnitrils bis zu ca. 3% Rückspaltungsprodukte wie Methylmercaptopropionaldehyd bzw. das entsprechende MMP-Cyanhydrin im Laufe der Reaktion bilden können. Diese Produkte sind hochgradig unerwünscht, da sie zum einen einen entsprechenden Verlust an Methioninausbeute bedeuten und zum anderen zur Bildung von Oligomeren und weiteren Nebenprodukten führen, die in der Isolierungs- bzw. Reinigungsstufe des reinen Methioninprodukts am Ende des Verfahrens große Schwierigkeiten machen können, insbesondere in der Kristallisation. So können die Filtrierbarkeit des Endproduktes massiv beeinträchtigt werden, was zu einer Reduzierung der Raum-/Zeitausbeute führt. Außerdem nimmt der Anteil an auszuschleusender Mutterlauge dadurch deutlich zu und damit die Verluste an Wertstoff. Dabei beginnen bereits Anteile von größer als 0,2 Mol% Methylmercaptopropionaldehyd oder MMP-Cyanhydrin in der Amidstufe die Aufarbeitung deutlich zu stören und sind entsprechend unerwünscht.

### Aufgabe:

Die der vorliegenden Erfindung grundsätzlich zugrundeliegende Aufgabe war demzufolge die Bereitstellung eines möglichst einfachen chemischen Verfahrens zur Herstellung von D,L-Methionin, bei dem weniger harsche Bedingungen als in der klassischen Methode über das Hydantoin ermöglicht werden. Dabei sollte gleichzeitig der Zwangsanfall von Salzen als Koppelprodukt der klassischen Verfahren minimiert sowie damit einhergehende hohe Salzfrachten in Prozesskreislaufströmen vermieden werden. Eine besondere Aufgabe in diesem Zusammenhang war es, ein Verfahren zur möglichst salzarmen Herstellung von D,L-Methioninamid bereitzustellen, welches dann durch Kopplung mit einem Verfahren zur Neutralverseifung von D,L-Methioninamiden zu möglichst salzarmen Gesamtverfahren zur Herstellung von D,L- Methionin ergänzbar ist, und bei dem gleichzeitig möglichst weniger als 0,2 % Methylmercaptopropionaldehyd sowie möglichst keinerlei MMP-Cyanhydrin als Rückspaltungsprodukte gebildet werden.

### Lösung:

Die Erfinder haben überraschenderweise herausgefunden, dass es im Gegensatz zu den bisherigen Veröffentlichungen, bei denen für die carbonylkatalysierte basische Hydrolyse von Aminonitril starke Alkalibasen wie Natronlauge oder Kalilauge äquivalent verwendet werden, im Falle von Methioninnitril auf eine genaue Auswahl der Base und der Bedingungen ankommt, um die oben genannten negativen technischen Effekte zu vermeiden. Es wurde gefunden, dass eine spezifische Menge von KOH bei bestimmten Temperatur- und Konzentrationsverhältnissen zu einer deutlichen Verringerung der Rückspaltungsprodukte führt. Dies ist überraschenderweise nur mit Kaliumhydroxid der Fall, nicht jedoch mit Natrium-oder Lithiumhydroxid.
Die oben genannten Aufgaben wurden somit erfindungsgemäß gelöst durch das Bereitstellen eines Verfahrens zur Herstellung von Methioninamid durch Hydrolyse von Methioninnitril in einem wässrigen Reaktionsgemisch enthaltend 0,5 bis 1,5 Moleq eines Ketons als carbonylhaltigen Katalysator und von 0,03 bis 0,10 Moleq, vorzugsweise von 0,04 bis 0,09 Moleq, besonders bevorzugt von 0,05 bis 0,09 Moleq KOH als basischen Katalysator jeweils bezogen auf 1 Moleq Methioninnitril.

Die erfindungsgemäße Menge von 0,03 bis 0,10 Moleq, vorzugsweise von 0,04 bis 0,09 Moleq, besonders bevorzugt von 0,05 bis 0,09 Moleq KOH als basischer Katalysator führt im erfindungsgemäßen Verfahren in überraschender Weise zu vollständigem Umsatz bei sehr hohen Selektivitäten; also in praktisch quantitativen Ausbeuten zu Methioninamid und kleineren Anteilen von 2,2-Dimethyl-5-(2'-methylthioethyl)-imidazolidin-4-on (IM2) und 2,5-Di-(2'-methylthioethyl)-imidazolidin-4-on (IM1), den direkten Methioninvorstufen, wobei gleichzeitig nur minimale Rückbildungsraten von MMP bzw. MMP-Cyanhydrin von < 0,2 % erhalten werden.

Im Gegensatz dazu sind die Umsätze mit Lithium-, Calcium- bzw. Magnesiumhydroxid als basischer Katalysator in der gegebenen Zeit nicht mehr vollständig, womit auch gleichzeitig eine geringere Selektivität durch eine erhöhte Rate der Nebenreaktionen verbunden ist (Vergleichsbeispiele 9-11). Selbst das in Verfahren des Stands der Technik wie z.B. gemäß WO9408957 verwendete Natriumhydroxid zeigt bei gleichen molaren Konzentrationen schlechtere Ergebnisse im Vergleich zu Kaliumhydroxid, wie der Vergleich von Beispiel 2 und 8 bzw. 6 und 7 deutlich macht. Insbesondere treten hier z.T. deutlich höhere Anteile als 0,2 % an MMP oder MMP-Cyanhydrin auf (>3%), so dass damit die erfindungsgemäße Aufgabe nicht mehr gelöst werden kann (vgl. insbesondere Tabelle 1).

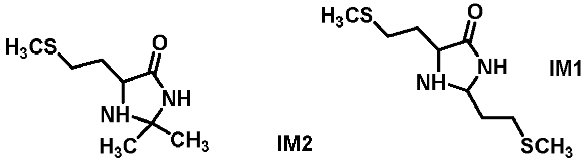

Diese vergleichsweise geringen Mengen an Hydroxidbase von maximal 0,10 Moleq, wie insbesondere 0,03, 0,04 oder 0,05 Moleq (entsprechend 3, 4 oder 5 Mol%; Beispiele 4, 5 und 2) haben den Vorteil, dass sie nach der Reaktion durch eine entsprechend geringe Mengen an Säure zum entsprechenden Salz neutralisiert werden können und dann leicht abgetrennt werden können. Dies gewährleistet eine nahezu salzfreie Herstellung von D,L-Methioninamid und bei Kombination mit einer Neutralverseifung des entstandenen Amids z.B. mit Hilfe von TiO₂-haltigen Hydrolysekatalysatoren eine nahezu salzfreie Route zum Methionin ausgehend von Methylmercaptopropionaldehyd. Zum anderen sind sie ausreichend, um praktisch vollständigen Umsatz bei vergleichsweise niedrigen Temperaturen zu gewährleisten. Dies war überraschend, weil im Stand der Technik mit höheren Äquivalentmengen an Basen wie z.B. 20 Mol% NaOH gearbeitet worden ist (vgl.EP0228938, WO94/08957 A1).
Bevorzugt wird dabei ein Verfahren bei dem als Keton-Katalysator, Aceton, Butanon, 2- oder 3-Pentanon, 2-oder 3-Hexanon, Cyclopentanon, Cyclohexanon oder 4-Piperidon eingesetzt wird. Alle diese Carbonylverbindungen sind kommerziell problemlos erhältlich. Der Preis ist dabei sekundär, weil die Carbonylverbindungen als Katalysatoren eingesetzt werden, d.h. bis auf kleinere Verluste nicht verbraucht sondern zurückgewonnen und wiederverwendet werden.

Des Weiteren bevorzugt wird dabei die Hydrolyse des Methioninnitrils bei einer Temperatur von 20 bis 45°C, vorzugsweise von 22 bis 40°C, besonders bevorzugt von 25 bis 38°C durchgeführt, weil so die maximale Ausbeute bei geringsten Nebenproduktanteilen und vergleichsweise kurzen Reaktionszeiten erhalten wird.

Darüber hinaus bevorzugt wird die Hydrolyse des Methioninnitrils bei einer Methioninnitrilkonzentration von 10 bis 30 Gew.%, vorzugsweise von 15 bis 25 Gew.% bezogen auf die Gesamtmasse des Reaktionsgemisches durchgeführt. Dies trägt gleichermaßen bei sowohl zu einer ausreichend hohen Umsatzgeschwindigkeit wie auch einer ausreichend guten Rührbarkeit bzw. Pumpbarkeit in den entsprechenden Reaktoren wie z.B. Rührkesseln oder Strömungsrohren bei.

Ebenfalls Gegenstand der Erfindung ist ein Gesamtverfahren zur Herstellung von Methioninamid gekennzeichnet durch folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltenden Reaktionsgemisch,
b. optional wenigstens teilweise Abtrennung von restlichem Ammoniak aus dem Reaktionsgemisch aus a.,
c. Hydrolyse des Methioninnitrils zum Methioninamid im Reaktionsgemisch aus a. oder b. gemäß dem oben dargestellten erfindungsgemäß bereitgestellten Verfahren und
d. optional Abtrennung des Ketons und/oder des basischen Katalysators aus dem Reaktionsgemisch aus c.

Ein ebenfalls mit dem vorstehend genannten Verfahren zur Herstellung von Methioninamid eng verknüpfter Erfindungsgegenstand ist ein Verfahren zur Herstellung von Methionin dadurch gekennzeichnet, dass man das gemäß Schritt c. oder d. erhaltene Methioninamid in einem Schritt e. weiterhydrolysiert zum Methionin in Gegenwart eines sauren, basischen oder neutralen Katalysators. Durch die Anwendung eines neutralen Katalysators wird ein insgesamt nahezu salzfreies Verfahren zur Herstellung von Methionin bereitgestellt, was von erheblichem Vorteil ist, wie eingangs dargestellt. Das dabei primär entstehende Ammoniummethioninat kann anschließend thermisch gespalten werden in Methionin, das dabei als Kristallisat anfällt, und flüchtigen Ammoniak, der leicht abgetrennt und z.B. in die Methioninnitril-Synthesestufe zurückgeführt werden kann.

Bei der o.g. Umsetzung gemäß Schritt a. werden vorzugsweise 1 bis 10 Moleq Ammoniak, mehr bevorzugt 2 bis 8 Moleq Ammoniak und besonders bevorzugt 4 bis 7 Moleq Ammoniak eingesetzt bezogen auf die molare Summe aus Methylmercaptopropionaldehyd und MMP-Cyanhydrin. Insbesondere die Ammoniaküberschüsse gewährleisten, dass nur sehr wenig, nämlich <0,1% des dimeren Iminodinitrilproduktes als Nebenprodukt entstehen (siehe Beispiel 1).

Bei der Umsetzung gemäß Schritt a. wird vorzugsweise wässriger Ammoniak in einer Konzentration von 25 bis 80 Gew.-%, mehr bevorzugt von 30 bis 60 Gew.-% eingesetzt, In der Regel genügt es mit ca. 32 Gew.% Ammoniak zu arbeiten, was den zusätzlichen Vorteil bietet, dass der eingesetzte Ammoniak dabei zunächst noch bei Normaldruck zu handhaben ist. Auch die höhere Ammoniakkonzentration trägt zu einer Verringerung der Iminodinitrilbildung bei.

Die Abtrennung von restlichem Ammoniak gemäß Schritt b. erfolgt vorzugsweise bei einem Druck von 0,001 bis 1 bar und einer Temperatur von 20 bis 100 °C, besonders bevorzugt von 0,01 bis 0,9 bar und einer Temperatur von 25 bis 90°C. Dabei wird auch immer anteilig Wasser mit abgetrennt.

Unter ökonomischen wie ökologischen Gesichtspunkten ist es vorteilhaft, wenn das gemäß Schritt d. abgetrennte Keton zur Hydrolyse des Methioninnitrils in Schritt c. zurückgeführt wird. Dies kann unmittelbar oder wenn zweckmäßig nach einer Zwischenreinigung z.B. durch Destillation geschehen.

Bei der Hydrolyse des Methioninamids zum Methionin gemäß Schritt e. wird im Falle der Verwendung eines sauren Katalysators vorzugsweise eine starke Mineralsäure, bevorzugt Salzsäure, Schwefelsäure oder Phosphorsäure verwendet, welche allesamt sehr wirkungsvoll und kostengünstig erhältlich sind.

Im Falle der Verwendung eines basischen Katalysators bei der Hydrolyse des Methioninamids zum Methionin gemäß Schritt e. wird vorzugsweise eine Alkali- oder Erdalkali- Base verwendet, insbesondere NaOH, KOH, Mg(OH)₂, Ca(OH)₂ oder Ba(OH)₂. Vorteilhaft dabei ist die schnelle und vollständige Hydrolyse zum Methionin bei moderaten Temperaturen. Nachteilig ist dabei jedoch, dass das Methionin zunächst als Alkali- oder Erdalkalisalz anfällt, aus dem erst wieder durch Neutralisation mit Säure das Methionin freigesetzt werden muss, was zwangsläufig wiederrum zu einem Salzanfall führt. Vorteilhaft ist die basische Hydrolyse dagegen vor allem dann, wenn sowieso ein Alkali- oder Erdalkalimethioninat als Produktform gewünscht ist, da diese dann auf direktem Weg als Endprodukt erhältlich ist.

Als neutraler Katalysator bei der Hydrolyse des Methioninamids gemäß Schritt e. werden vorzugsweise Titandioxide verwendet, welche vorteilhafterweise kein nennenswertes Leaching-Verhalten aufweisen, jedoch in effektiver Weise den abschließenden Hydrolyseschritt katalysieren. Die Verwendung von neutralen Katalysatoren ist hier besonders bevorzugt, weil nur so ein insgesamt salzarmes Verfahren zur Herstellung von Methionin bereitgestellt werden kann. Dabei bevorzugt verwendet werden kann beispielsweise ein Katalysator von Sachtleben (Hombikat F01), der einen pH-Wert von ca. 6, eine spezifische Oberfläche von 350 m²/g, einen Titandioxidgehalt (geglüht) von 88 % sowie bedingt durch den Produktionsprozesses noch ca. 0,5 % Schwefelsäure aufweist. Diese sind auch auf kommerziellem Weg leicht verfügbar, würden aber auch nicht verloren, sondern nach ihrer Verwendung zurückgewonnen und recycliert.

Dabei bevorzugt wird ein Verfahren, dadurch gekennzeichnet, dass vor der Hydrolyse des Methioninamids mit einem neutralen Katalysator gemäß Schritt e. der vorhandene basische Katalysator (KOH) mit Säure, vorzugsweise Schwefelsäure, Phosphorsäure, Salzsäure, Ameisensäure oder Kohlensäure zum entsprechenden Salz neutralisiert und ggf. abgetrennt wird. Bei diesem Salz handelt es sich je nach verwendeter Säure um Kaliumchlorid, Kaliumcarbonat, Kaliumhydrogencarbonat oder Kaliumsulfat, Kaliumhydogensulfat, Kaliumphosphat, Kaliumhydogenphosphat oder Kaliumdihydogenphosphat

Die Salzabtrennung kann auch erst nach der Hydrolyse des Methioninamids und dann vorteilhaft durch Auskristallisation des Methionins erfolgen, wobei die leichter als Methionin wasserlöslichen Salze des Kaliums in der Mutterlauge verbleiben, die entweder aufgearbeitet werden kann, so dass daraus wiederum durch Kristallisation das betreffende Salz erhalten werden kann, oder direkt als Flüssigprodukt oder als Festprodukt nach Eindampfung, insbesondere nach Sprühgranulation an die Düngemittelindustrie oder auch an Anwender abgegeben werden kann zur Verwendung als Flüssig- oder Feststoffdünger. Da die Kaliumsalze als Kaliumdünger begehrt sind, ist es hier erfindungsgemäß gelungen, noch ein weiteres wertvolles Produkt bereitzustellen. Da auf der anderen Seite nur 0,03 bis 0,10 Moleq der entsprechenden Salze bezogen auf das Methioninprodukt anfallen, halten sich bedingt durch die relativ kleinen Mengen die typischen Nachteile einer Koppelproduktion in Grenzen und es ist auf diese Weise auch gelungen, ein sehr ökonomisches wie auch ökologisch vertretbares Verfahren zur Methioninherstellung bereitzustellen.

Bevorzugt wird die Hydrolyse des Methioninamids zum Methionin gemäß Schritt e. bei Temperaturen von 80-180°C, vorzugsweise von 100-140°C durchgeführt. Dadurch wird insbesondere eine vollständige Umsetzung erreicht und damit ein qualitativ hochwertiges von Anfang an sehr reines Endprodukt.

### Beispiele:

### Analytische Methoden

Die chromatographischen Untersuchungen (MMP-Cyanhydrin, MMP, MMP-AN, IM1, IM2 Methionin, Methioninamid) wurden mittels HPLC der Firma JASCO an einer RP-18 Säule (250 x 4,6 mm; 5 µm) mit anschließender UV Detektion bei 210 nm durchgeführt. Als Laufmittel diente ein phosphorsaures Acetonitril-Wasser-Gemisch (3,3 g H₃PO₄, 6,8 g Acetonitril, 89,9 g H₂O). Bei einem Fluss von 1 mL/min wurden 10 µL der jeweiligen Probelösung (50 mg Probe in 25 mL H₂O) injiziert. Die Kalibrierung erfolgte im Vorfeld durch die Injektion geeigneter Kalibrierlösungen, die Auswertung erfolgte durch Peakflächenvergleich mit externen Standards. Die Durchführung der Standardmethode ist dem Fachmann bekannt.

### Beispiel 1: Synthese von Methioninnitril ausgehend von MMP-Cyanhydrin

64,0 g MMP-Cyanhydrin (90% in Wasser, 0,439 mol, 1 Moleq) wurde im 1 L-Reaktor bei 40 °C vorgelegt und unter Rühren mit 168,0 g Ammoniak (32 Gew.% in Wasser, 7 Moleq, 3,107 mol) versetzt (MMP-Cyanhydrin-Konzentration 25 Gew.%). Die beigefarbene und trübe, aber gut rührbare Emulsion wurde auf 50 °C temperiert und 30 min gerührt (ein Überschreiten von 55 °C durch die Exothermie der Reaktion wurde dabei vermieden). Die erhaltene klare, hellgelbe Lösung zeigte mittels HPLC-Analytik eine 100%-ige Umsetzung des MMP-Cyanhydrins bei 98,8%-iger Selektivität zu Methioninnitril-Äquivalenten (Methioninnitril (0,422 mol) und Methioninamid (0,011 mol)). In Spuren war eine Bildung des Iminodinitrils 2,2'-Bis-(2-methylmercaptoethyl)-iminodiacetonitril (DN1, <0,1%) zu beobachten.

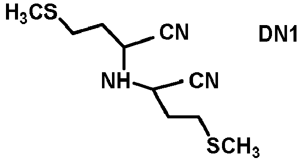

### Beispiel 2: Direkte Umsetzung des erhaltenen Methioninnitrils zu Methioninamid

Die im ersten Reaktionsschritt erhaltene Reaktionslösung enthaltend 54,9 g Methioninnitril (0,422 mol), 1,6 g Methioninamid (0,011 mol; Methioninnitril und Methioninamid bilden zusammen 1 Moleq), 45,4 g Ammoniak (2,67 mol, 6 Moleq) und 120 g Wasser wurde auf 35°C abgekühlt und nacheinander mit 43 g Wasser, 25 g Aceton (1 Moleq, 0,43 mol) und 13 g KOH-Lösung (10 Gew.% in Wasser, 0,05 Moleq, 0,023 mol) versetzt (Methioninnitril-Konzentration 17 Gew.%). Das Reaktionsgemisch wurde unter Rühren 1,5 Stunden bei 35°C gehalten. Die erhaltene klare, gelbe Lösung wies mittels HPLC-Analytik eine 100%-ige Umsetzung des Methioninnitrils bei 98,5%-iger Selektivität zu Methioninamid-Äquivalenten auf. Dabei entstanden die Methioninamid-Äquivalente Methioninamid (0,418 mol) und 0,01 mol des entsprechenden Imidazolidinons aus der Reaktion von Methioninnitril mit Aceton (IM2), welches bei Temperaturerhöhung Aceton freisetzt und sich demnach wie Methioninamid verhält. Der pH-Wert der Reaktion fiel dabei von pH 13,5 zu Beginn auf pH 13 am Ende ab. In Spuren war eine weitere Imidazolidinon-Komponente (IM1) aus der Reaktion mit MMP sowie das beschriebene Iminodinitril DN1 samt Folgeprodukte (<0,1%), bei denen die beiden Nitril-Funktionen vollständig der zum Teil zu den entsprechenden Amiden oder Carbonsäuren umgesetzt wurden, zu beobachten. Die Rückbildung zu MMP wurde in Höhe von <0,2% nachgewiesen, eine Rückreaktion des Methioninnitrils zu MMP-CN wurde nicht detektiert.
Über die beiden Stufen ergibt sich somit ausgehend von 0,439 mol MMP- Cyanhydrin eine Ausbeute an Methioninamid -Äquivalenten von 97,3% d.Th. (0,428 mmol).

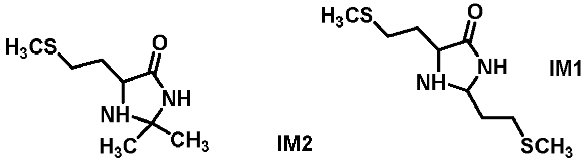

### Beispiele 3 - 12: Direkte Umsetzung des erhaltenen Methioninnitrils zu Methioninamid mit verschiedenen Hydroxidbasen und -konzentrationen und Aceton als Carbonylkatalysator

Wie in Beispiel 1 beschrieben wurde eine Reaktionslösung enthaltend 54,9 g Methioninnitril (0,422 mol,), 1,6 g Methioninamid (0,011 mol; Methioninnitril und Methioninamid bilden zusammen 1 Moleq), 45,4 g Ammoniak (2,67 mol, 6 Moleq) und 120 g Wasser hergestellt, auf 35 °C abgekühlt und dann nacheinander mit 43 g Wasser, 25 g Aceton (1 Moleq, 0,43 mol) sowie die jeweils in Tabelle 1 angegebene Menge der jeweils angegebenen Hydroxidbase zugegeben (z.B. 5 mol% einer NaOH- oder KOH-Lösung (10 Gew.% in Wasser;.0,023 mol) versetzt (Methioninnitrilkonzentration 17 Gew.%).
Das Reaktionsgemisch wurde 1,5 Stunden bei 35 °C gerührt. Die erhaltene klare, gelbe Lösung wurde mittels HPLC analysiert und der Umsatz des Methioninnitrils sowie Selektivität zu Methioninamid -Äquivalenten (Methioninamid, Kalium- bzw. Natriummethioninat sowie IM2) bestimmt (Auswertung mit Gesamtausbeute siehe Tabelle 1).

**Tabelle 1: Hydrolyse von Methioninnitril mit verschiedenen Basen und Aceton als Carbonylkatalysator**

| **Beispiel** | **Base** | **MOH** | **Ausbeute** | **Umsatz** | **Selektivtät** | **MMP- Bildung** | **MMP-CN- Bildung** |
|---|---|---|---|---|---|---|---|
| | **MOH** | **[Mol%]** | **[%]** | **[%]** | **[%]** | **[%]** | **[%]** |
| 3 (Vergleich) | KOH | 1 | 45 | 94 | 48 | 10 | 5 |
| 4 | KOH*) | 3 | 70 | 70 | 99 | <0,2 | 0 |
| 5 | KOH | 4 | 88 | 99,5 | 88 | <0,2 | 0 |
| 2 | KOH | 5 | 98,5 | 100 | 98,5 | <0,2 | 0 |
| 6 | KOH | 10 | 98 | 100 | 98 | <0,1 | 0 |
| 7 (Vergleich) | NaOH | 10 | 92,7 | 100 | 92,7 | 3 | 0 |
| 8 (Vergleich) | NaOH | 5 | 90,5 | 100 | 90,5 | 3 | 0,6 |
| 9 (Vergleich) | LiOH | 5 | 87 | 94 | 92 | 2 | <0,2 |
| 10 (Vergleich) | Ca(OH)₂ | 2,5 | 67 | 82 | 81 | 10 | 5 |
| 11 (Vergleich) | Mg(OH)₂ | 2,5 | 38 | 42 | 89 | 5 | 2 |
| 12 (Vergleich) | ohne | - | 34 | 92 | 37 | 10 | 10 |

| | | | | | | | |
|---|---|---|---|---|---|---|---|
| *) In Beispiel 4 wurde abweichend von den anderen Beispielen in Tabelle 1 die MMP-AN-Lösung binnen 30 min. zu 3 Mol% (0,03 Moleq) einer verdünnten KOH-Lösung mit 1 Moleq Aceton in Wasser getropft und nach weiteren 60 min Reaktionszeit der Umsatz (70%), die Ausbeute (70%) und die Selektivität (>99%) bestimmt. | | | | | | | |

Überraschenderweise zeigte sich, dass bei Verwendung sehr kleiner Äquivalentmengen Kaliumhydroxid (1 Mol%) als basischer Katalysator bereits fast quantitative Umsätze (94%) erreicht werden. Die Selektivität lag bei den hier gewählten Bedingungen allerdings nur bei 48 % bei sehr hohen Rückspaltungsraten zu MMP bzw. MMP-Cyanhydrin von 10% bzw. 5%.

Etwas höhere Äquivalentmengen Kaliumhydroxid (3 bis 4 Mol%) als basischer Katalysator führen im hier gewählten Zeit- und Temperaturfenster allerdings teilweise bereits zu quantitativen Umsätzen (88 bis 99%) bei deutlich höheren Selektivitäten (77 bis 99%) bei erfindungsgemäß niedrigen Rückspaltungsraten zu MMP bzw. MMP-Cyanhydrin von < 0,2%.

Noch etwas höhere Äquivalentmengen Kaliumhydroxid (5 bis 10 Mol%) als basischer Katalysator führen im hier gewählten Zeit- und Temperaturfenster schließlich zu quantitativen Umsätzen (100%) bei sehr hohen Selektivitäten (bis ≥99%) bei erfindungsgemäß niedrigen Rückspaltungsraten zu MMP bzw. MMP-Cyanhydrin von <0,2%.

Die Umsätze sind mit Lithium-, Calcium- bzw. Magnesiumhydroxid als basischer Katalysator in der gegebenen Zeit nicht mehr vollständig, womit auch gleichzeitig eine geringere Selektivität durch eine erhöhte Rate der Nebenreaktionen verbunden ist (Vergleichsbeispiele 11 - 13). Selbst das in Verfahren des Stands der Technik wie z.B. gemäß WO9408957 verwendete Natriumhydroxid zeigt bei gleichen molaren Konzentrationen schlechtere Ergebnisse mit deutlich über 0,2 % liegenden Rückspaltungsraten für MMP oder MMP-Cyanhydrin im Vergleich zu Kaliumhydroxid, wie der Vergleich von Beispiel 2 und 8 bzw. 6 und 7 deutlich macht.

### Beispiele 13-15: Hydrolyse von Methioninnitril mit KOH und Aceton als Carbonylkatalysator bei verschiedenen Temperaturen bzw. Konzentrationen

Wie in Beispiel 1 beschrieben wurde eine Reaktionslösung enthaltend 54,9 g Methioninnitril (0,422 mol,), 1,6 g Methioninamid (0,011 mol;Methioninnitril und Methioninamid bilden zusammen 1 Moleq), 45,4 g Ammoniak (2,67 mol, 6 Moleq) und 120 g Wasser hergestellt, auf 35 °C abgekühlt und dann nacheinander mit 43 g Wasser, 25 g Aceton (1 Moleq, 0,43 mol) sowie 5 mol% einer KOH-Lösung (10 Gew.% in Wasser;.0,023 mol) versetzt (Methioninnitrilkonzentration 17 Gew.%).
Das Reaktionsgemisch wurde 1,5 Stunden bei der in Tabelle 2 angegebenen Temperatur gerührt. Die erhaltene klare, gelbe Lösung wurde mittels HPLC analysiert und der Umsatz des Methioninnitrils sowie Selektivität zu Methioninamid-Äquivalenten (Methioninamid, Kaliummethioninat sowie IM2) bestimmt (Auswertung mit Gesamtausbeute siehe Tabelle 2).

**Tabelle 2: Hydrolyse bei verschiedenen Temperaturen bzw. Konzentrationen**

| **Beispiel** | **Temperatur** | **Ausbeute [%]** | **Umsatz [%]** | **Selektivität [%]** | **MMP-Bildung [%]** | **MMP-CN- Bildung [%]** |
|---|---|---|---|---|---|---|
| 13 | 25°C | ≥ 99,5 | 100,0 | ≥ 99,5 | <0,1 | 0 |
| 2 | 35°C | 98,5 | 100 | 98,5 | <0,2 | 0 |
| 14 | 45°C | 95,0 | 100 | 95,0 | <0,2 | 0 |
| 15 | 35°C, mit 20 Gew% MMP-AN | ≥ 99,5 | 100 | ≥ 99,5 | <0,2 | 0 |

Der Vergleich der Ergebnisse zeigt, dass sich Temperaturen von 25 bis 35 °C besonders vorteilhaft auf Umsatz, Selektivität und Ausbeute auszuwirken scheinen. Auch zeigt der Vergleich von Beispiel 2 und 15, dass eine Erhöhung der Methioninnitril-Konzentration sich entsprechend vorteilhaft auswirkt.

## Patentansprüche

1. Verfahren zur Herstellung von Methioninamid durch Hydrolyse von Methioninnitril in einem wässrigen Reaktionsgemisch enthaltend 0,5 bis 1,5 Moleq eines Ketons als carbonylhaltiger Katalysator und von 0,03 bis 0,10 Moleq, vorzugsweise von 0,04 bis 0,09 Moleq, besonders bevorzugt von 0,05 bis 0,09 Moleq KOH als basischer Katalysator jeweils bezogen auf 1 Moleq Methioninnitril.

2. Verfahren gemäß Anspruch 1, **dadurch gekennzeichnet, dass** es sich bei dem Keton um Aceton, Butanon, 2- oder 3-Pentanon, 2-oder 3-Hexanon, Cyclopentanon, Cyclohexanon oder 4-Piperidon handelt.

3. Verfahren gemäß Anspruch 1 oder 2, **dadurch gekennzeichnet, dass** die Hydrolyse des Methioninnitrils bei einer Temperatur von 20 bis 45°C, vorzugsweise von 22 bis 40°C, besonders bevorzugt von 25 bis 38°C durchgeführt wird.

4. Verfahren gemäß einem der Ansprüche 1 bis 3, **dadurch gekennzeichnet, dass** die Hydrolyse des Methioninnitrils bei einer Methioninnitrilkonzentration von 10 bis 30 Gew.%, vorzugsweise von 15 bis 25 Gew.% bezogen auf die Gesamtmasse des Reaktionsgemisches durchgeführt wird

5. Verfahren zur Herstellung von Methioninamid **gekennzeichnet durch** folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltenden Reaktionsgemisch,
b. optional wenigstens teilweise Abtrennung von restlichem Ammoniak aus dem Reaktionsgemisch aus a.,
c. Hydrolyse des Methioninnitrils zum Methioninamid im Reaktionsgemisch aus a. oder b. gemäß einem der Ansprüche 1 bis 4 und
d. optional Abtrennung des Ketons und/oder des basischen Katalysators aus dem Reaktionsgemisch aus c.

6. Verfahren zur Herstellung von Methionin **dadurch gekennzeichnet, dass** man das gemäß Anspruch 5 Schritt c. oder d. erhaltene Methioninamid in einem Schritt e. weiterhydrolysiert zum Methionin in Gegenwart eines sauren, basischen oder neutralen Katalysators.

7. Verfahren gemäß Anspruch 5 oder 6, **dadurch gekennzeichnet, dass** bei der Umsetzung gemäß Schritt a. 1 bis 10 Moleq Ammoniak, vorzugsweise 2 bis 8 Moleq Ammoniak und besonders bevorzugt 4 bis 7 Moleq Ammoniak eingesetzt werden bezogen auf die molare Summe aus Methylmercaptopropionaldehyd und MMP-Cyanhydrin.

8. Verfahren gemäß einem der Ansprüche 5 bis 7, **dadurch gekennzeichnet, dass** bei der Umsetzung gemäß Schritt a. wässriger Ammoniak in einer Konzentration von 25 bis 80 Gew.-%, mehr bevorzugt von 30 bis 60 Gew.-% eingesetzt wird.

9. Verfahren gemäß einem der Ansprüche 5 bis 8, **dadurch gekennzeichnet, dass** die Abtrennung von restlichem Ammoniak gemäß Schritt b. bei einem Druck von 0,001 bis 1 bar und einer Temperatur von 20 bis 100 °C, vorzugsweise von 0,01 bis 0,9 bar und einer Temperatur von 25 bis 90°C erfolgt.

10. Verfahren nach einem der Ansprüche 5 bis 9, **dadurch gekennzeichnet, dass** das gemäß Schritt d. abgetrennte Keton zur Hydrolyse des Methioninnitrils in Schritt c. zurückgeführt wird.

11. Verfahren gemäß einem der Ansprüche 6 bis 10, **dadurch gekennzeichnet, dass** bei der Hydrolyse des Methioninamids gemäß Schritt e. als saurer Katalysator eine starke Mineralsäure, vorzugsweise Salzsäure, Schwefelsäure oder Phosphorsäure verwendet wird.

12. Verfahren gemäß einem der Ansprüche 6 bis 10, **dadurch gekennzeichnet, dass** bei der Hydrolyse des Methioninamids gemäß Schritt e. als basischer Katalysator ein Alkali- oder Erdalkali- Base, vorzugsweise NaOH, KOH, Mg(OH)₂, Ca(OH)₂ oder Ba(OH)₂ verwendet wird.

13. Verfahren gemäß einem der Ansprüche 6 bis 10, **dadurch gekennzeichnet, dass** bei der Hydrolyse des Methioninamids gemäß Schritt e. als neutraler Katalysator Titandioxid verwendet wird.

14. Verfahren gemäß einem der Ansprüche 6 bis 10 oder 13, **dadurch gekennzeichnet, dass** der basische Katalysator vor der Hydrolyse des Methioninamids mit einem neutralen Katalysator gemäß Schritt e. mit Säure, vorzugsweise Schwefelsäure, Phosphorsäure, Salzsäure, Ameisensäure oder Kohlensäure zum entsprechenden Salz neutralisiert und ggf. abgetrennt wird.

15. Verfahren nach einem oder mehreren der Ansprüche 11 bis 14, **dadurch gekennzeichnet, dass** die Hydrolyse des Methioninamids gemäß Schritt e. bei Temperaturen von 80-180°C, vorzugsweise von 100-140°C durchgeführt wird.
